Europäisches Patentamt

European Patent Office

Office européen des brevets

(11) Veröffentlichungsnummer: **0 343 454**
**A2**

## (12) EUROPÄISCHE PATENTANMELDUNG

(21) Anmeldenummer: 89108578.9

(22) Anmeldetag: 12.05.89

(51) Int. Cl.⁴: **C07C 43/04 , C07C 41/09 , C07C 41/34**

(30) Priorität: 26.05.88 DE 3817816

(43) Veröffentlichungstag der Anmeldung:
29.11.89 Patentblatt 89/48

(84) Benannte Vertragsstaaten:
AT BE CH DE ES FR GB GR IT LI LU NL SE

(71) Anmelder: **Union Rheinische Braunkohlen Kraftstoff Aktiengesellschaft**
**Ludwigshafener Strasse o. Nr. Postfach 1663**
**D-5047 Wesseling(DE)**

(72) Erfinder: **Meisenburg, Ewald**
**Höhenring 60**
**D-5357 Swisstal-Heimerzheim(DE)**
Erfinder: **König, Gerhard, Dr.**
**Dieselstrasse 14**
**D-5047 Wesseling(DE)**

(54) Verfahren zur Herstellung von Dimethylether.

(57) Die Erfindung betrifft ein in eine Methanolsynthese integriertes Verfahren zur Herstellung von Dimethylether durch katalytische Dehydratisierung von Methanol über einem $\gamma$-$Al_2O_3$-Katalysator, der bevorzugt geringe Mengen $SiO_2$ enthält und Reinigung des Deyhdratisierungsprodukts durch Zuführung desselben in eine Destillationskolonne zur Gewinnung von reinem Dimethylether.

EP 0 343 454 A2

## Verfahren zur Herstellung von Dimethylether

Die Erfindung betrifft ein in eine Methanol-Syntheseanlage integriertes Verfahren zur Herstellung von Dimethylether durch katalytische Dehydratisierung von Methanol ohne vorherige Abtrennung des im Methanolreaktor nicht umgesetzten Synthesegases.

Bis zur Entwicklung der Methanol-Niederdruck-Synthese-Verfahren wurde Dimethylether als Nebenprodukt der Methanol-Hochdruck-Synthese in einer Menge von etwa 2 - 5 Gew.-% erhalten, bezogen auf die Gesamtmenge des Synthesereaktor-Ausgangsprodukts und bei der Gewinnung von Reinmethanol destillativ aus dem weitere Verunreinigungen enthaltenden Vorlauf abgetrennt.

Nach Einführung der Niederdruck-Methanolsynthese-Verfahren, bei denen keine nennenswerten Mengen an Dimethylether anfallen, wurden Syntheseverfahren zur Herstellung von Dimethylether durch katalytische Dehydratisierung von Methanol entwickelt. Zahlreiche Verfahren sind in der Patentliteratur beschrieben worden. So lassen sich nach DE-PS 680 328 aliphatische Ether durch Erhitzen von Alkoholen in Gegenwart von Zinkchlorid erzeugen.

Weitere geeignete Katalysatoren zur Herstellung von Ethern aus Alkoholen sind gemäß GB-PS 332 756, GB-PS 350 010, GB-PS 403 402, US-PS 1 873 537 und FR-PS 701 335 Eisenchlorid, Kupfersulfat, Zinnchlorid, Manganchlorid, Aluminiumchlorid und -sulfat, Chromsulfat, Alaune, Thorium-Verbindungen, Aluminiumoxid, Titanoxid, Bariumoxid, Silicagel oder Aluminiumphosphat.

In "Industrial and Engineering Chemistry", Vol. 41, No. 12, S. 2928, 1949 wird die Verwendung von Bauxiten mit $SiO_2$-Gehalten von 4,40 - 13,99 Gew.-% beschrieben.

In US-PS 3 036 134 wird ein Aluminiumsilikat-Katalysator offenbart, zur Herstellung von Dimethylether aus Methanol, der ein $Al_2O_3$ : $SiO_2$ - Verhältnis von 1 Teil zu 1,35 bis 0,3 Teilen aufweist.

Auch die Synthese vom Dimethylether direkt aus Synthesegas (CO + $H_2$) ist beschrieben worden (DE-PS 23 62 944, DE-PS 27 57 788 und DE-PS 32 20 547).

Als technisch wichtigste Katalysatoren haben sich gemäß DE-PS 28 18 831, DE-OS 32 01 155, EP-A 0 099 676 und EP-A 0 124 078 insbesondere Aluminiumoxid- und Aluminiumsilikat-Katalysatoren mit und ohne Dotierung durchgesetzt.

In DE-PS 28 18 831 wird ein Katalysator zur Herstellung von Dimethylether offenbart, der jedes beliebige Aluminiumoxid-Grundmaterial mit genügend großer Oberfläche enthalten kann, in dem zusätzlich seltene Erden mit 1 - 30 Gew.-% $SE_2O_3$ vorhanden sind.

Schließlich ist in EP-A 0 099 676 ein Katalysator beschrieben, der 1 - 20 Gew.-% $SiO_2$, vorzugsweise 1 - 10 Gew.-% $SiO_2$ und noch bevorzugter 6 Gew.-% $SiO_2$ enthält.

In diesen Synthese-Anlagen wird reines, spezifikationsgerechtes Methanol eingesetzt.

Eine Verbesserung der bekannten Verfahren wird in der vorliegenden Erfindung offenbart, durch ein Verfahren zur katalytischen Herstellung von Dimethylether aus Methanol über Dehydratisierungskatalysatoren bei einer Temperatur von 140 - 500 °C und einem Druck von 1 bis 350 bar und destillative Aufarbeitung des Dehydratisierungsprodukts, dadurch gekennzeichnet, daß das aus dem Methanolsynthesereaktor austretende Gemisch zumindest teilweise, ohne vorherige Abtrennung nicht umgesetzten Synthesegases und ohne Reinigung des erzeugten Methanols, in einem Dehydratisierungsreaktor zur Gewinnung von Dimethylether umgesetzt wird.

Erfindungsgemäß kann nach beliebigen Verfahren Synthesegas erzeugt werden und in Methanol-Syntheseanlagen eingesetzt werden. Im allgemeinen handelt es sich bei letzteren um sog. Niederdrucksyntheseanlagen, wie z.B. das Lurgi-Verfahren, das bekanntlich unter 100 bar arbeitet oder das ICI-Verfahren, das um 100 bar arbeitet. Das Methanol kann jedoch nach anderen Verfahren erzeugt werden, auch durch sog. Mitteldruckverfahren.

Niederdruckverfahren sind bevorzugt, da die Investitionskosten für die Folgeanlagen, also den Dehydratisierungsreaktor und die Waschapparatur niedriger sind als bei Anwendung höherer Drücke. Grundsätzlich kann das Methanol jedoch auch in Hochdruckanlagen erzeugt werden, wobei man Dehydratisierungs- und Waschvorrichtungen bevorzugt bei niedrigeren Drücken betreiben würde. Das Kreislaufgas ist dann durch einen Kompressor wieder auf Methanolsynthesedruck zu bringen.

Da Niederdruckmethanolanlagen im allgemeinen bei niedrigerer Temperatur arbeiten als Dehydratisierungsreaktoren, ist es von Vorteil das Einsatzgemisch in dem Dehydratisierungsreaktor in einem Wärmetauscher durch das Produktgemisch aus dem Dehydratisierungsreaktor aufzuheizen.

Übliche Temperaturen in Niederdruckmethanolreaktoren liegen beispielsweise bei 200 - 300 °C, während Dehydratisierungsreaktoren bevorzugt oberhalb 300 °C betrieben werden.

Das Einsatzprodukt in dem Dehydratisierungsreaktor besteht aus dem im Methanolreaktor erzeugten Rohmethanol und der nicht umgesetzten Synthesegasmenge (Kreislaufgas).

Nach Verlassen des Dehydratisierungsreaktors gelangt das Produktgemisch in eine im allgemeinen unter Druck arbeitende Waschvorrichtung, in welcher der erzeugte Dimethylether aus gewaschen wird, bevorzugt mit Methanol oder Methanol/Wasser-Gemischen als Waschflüssigkeit, wobei im oberen Teil der Waschvorrichtung das Kreislaufgas abgezogen wird. Letzteres gelangt wieder vor den Methanolreaktor, wo ihm Frischgas (Synthesegas) zugeführt wird. Auch Wasser kann erfindungsgemäß als Waschflüssigkeit eingesetzt werden.

Wird die Waschvorrichtung bei niedrigem Druck betrieben, so ist das Kreislaufgas durch einen Kompressor wieder auf Methanolsynthesedruck zu bringen.

Das erfindungsgemäße Verfahren erlaubt es, dem Bedarf angepaßt, nur einen Teil des Methanolreaktor-Ausgangsgemischs in dem Dehydratisierungsreaktor umzusetzen, während der andere Teil über eine By-pass-Leitung direkt zur Waschvorrichtung geführt werden kann. Dieser Teil kann auch in einem üblichen unter Methanolsynthese-Druck stehenden Abscheider geführt werden, in dem nach Kühlung das Methanol abgeschieden werden und aus diesem entspannt werden kann.

Jedoch auch die Waschvorrichtung selbst kann bei Fahrweisen als Abscheider dienen, bei denen nur Methanol oder Methanol und Dimethylether erzeugt werden. Wird in dem in Methanol-Syntheseanlagen üblichen Hochdruckabscheider nur Methanol abgeschieden, so kann dieses dimethyletherfreie Rohmethanol auch als Waschflüssigkeit in die Waschvorrichtung für Dimethylether eingesetzt werden.

Es ist auch eine Fahrweise möglich, das aus diesem Abscheider abgezogene Rohmethanol im Dehydratisierungsreaktor synthesegasfrei umzusetzen. Es kann auch reines aus der Destillation gewonnenes Methanol im Dehydratisierungsreaktor umgesetzt werden.

Erfindungsgemäß kann Methanolsynthese und Dehydratisierung auch in einem Reaktor, insbesondere in einem sog. Abschnittsreaktor durchgeführt werden. Grundsätzlich können solche Katalysatoren verwendet werden, die zur Methanolbildung führen und zusätzlich wasserabspaltende Eigenschaften besitzen. Es können auch Gemische von Methanolkatalysatoren und Dehydratisierungskatalysatoren eingesetzt werden.

Möglich ist auch eine Fahrweise, bei der der Dehydratisierungskatalysator dem Methanolkatalysator getrennt, jedoch im gleichen Reaktor, nachgeschaltet ist.

Im allgemeinen wird in diesem Fall die Dehydratisierung bei dem gleichen Druck betrieben wie die Methanolsynthese, während im Bereich der Dehydratisierung durch eine zusätzliche Beheizung auch bei höherer Temperatur gearbeitet werden kann.

Das erfindungsgemäße Verfahren ist daher außerordentlich flexibel.

Die flüssige Phase aus der Waschapparatur wird einer destillativen Aufarbeitung zugeführt. Diese kann beispielsweise so erfolgen, daß in einer ersten Kolonne unter Druck, zweckmässigerweise zwischen 1 - 50 bar, bevorzugt bei Drucken < 10 bar reiner Dimethylether an einem der oberen Destillationsböden abgezogen wird, wobei Abgase wie z.B. kleine Mengen an $H_2$ und CO über Kopf abgenommen und einer Waschanlage zugeführt werden, in der kleine Mengen an Dimethylether bevorzugt mit Methanol ausgewaschen werden.

In einer 2. Kolonne können die zwischen Dimethylether und Methanol siedenden Verunreinigungen abgezogen werden, während in einer 3. Kolonne reines Methanol gewonnen wird, das als solches aus der Anlage entnommen werden kann und den Waschanlagen oder dem Dehydratisierungsreaktor zugeführt werden kann.

Die Destillation kann jedoch auch in beliebiger anderer Weise erfolgen. So kann beispielsweise in der ersten Kolonne der reine Dimethylether abgezogen werden und an einem tieferen Boden zumindest ein Teil der Verunreinigungen, während in einer 2. Kolonne Methanol am Kopf abgezogen wird und höher als Methanol siedende Verunreinigungen an einem Seitenabzug.

Es ist beispielsweise auch eine destillative Aufarbeitung möglich, bei der in der ersten Kolonne der reine Dimethylether gewonnen wird, in der 2. Kolonne über Kopf die zwischen Dimethylether und Methanol siedenden Verunreinigungen abgezogen werden und reines Methanol an einem Seitenabzug.

Der Sumpf der 1. Kolonne kann auch als Waschflüssigkeit für die Waschvorrichtung zum Auswaschen des Dimethylethers verwendet werden. Als Katalysator kann im Dehydratisierungsreaktor grundsätzlich jeder geeignete, Wasser abspaltende Katalysator eingesetzt werden, also insbesondere saure Katalysatoren. Bevorzugt sind $\gamma$-$Al_2O_3$-Katalysatoren, insbesondere solche mit niedrigem $SiO_2$-Gehalt nämlich von 0,0001 - 0,5, bevorzugt 0,001 - 0,4 Gew.-% $SiO_2$.

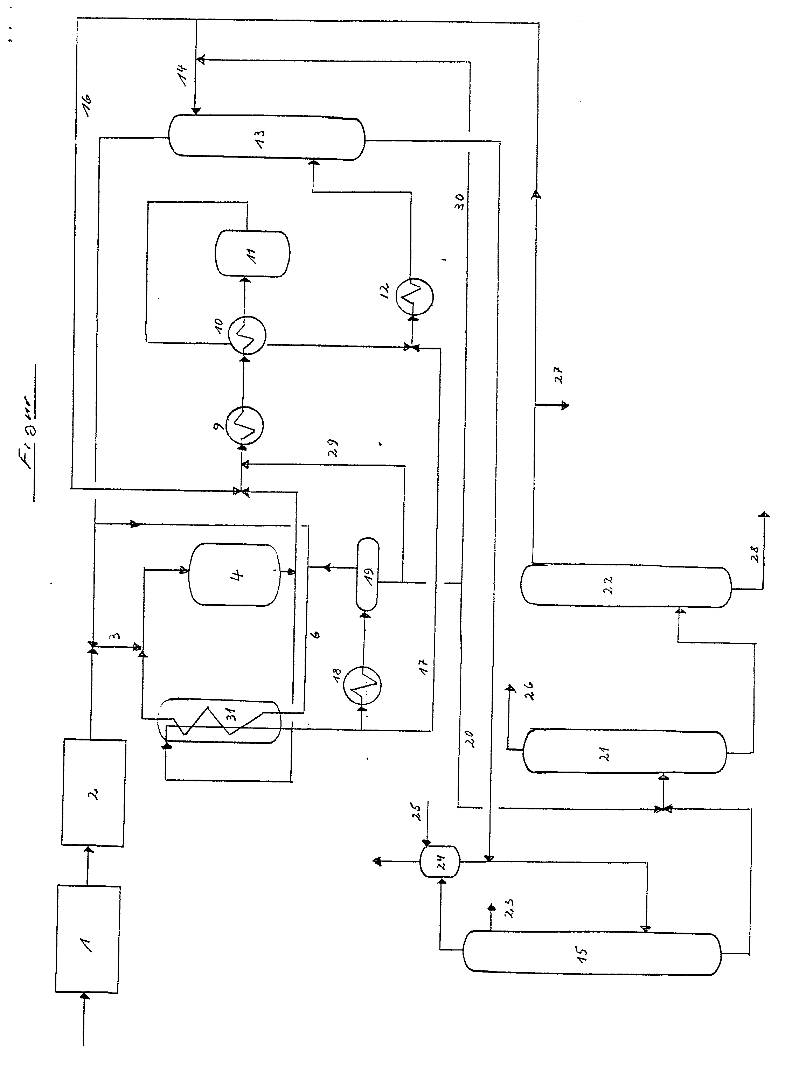

Mit Hilfe der Figur wird das erfindungsgemäße Verfahren beispielhaft näher erläutert.

In (1) wird Synthesegas erzeugt. Es kann sich hierbei um eine beliebige Anlage handeln wie z.B. eine Shell- oder Texaco-Öldruck-Vergasung, einen Wirbelschicht-Vergaser wie z.B. einen Hochtemperatur-Winklervergaser, einen Lurgi- oder Koppers-Totzek-Vergaser oder einen ICI-Steamreforming-Vergaser. In (2) wird das Gas $H_2S$- und CO-frei gewaschen. Auch hier sind beliebige Waschverfahren möglich. Beispielhaft sei das Rectisol-Verfahren genannt. Bekanntlich ist eine solche Wäsche im Falle der Synthesegaserzeugung nach dem ICI-Verfahren nicht erforderlich.Das Synthesegas gelangt über (3) in Methanolreaktor (4). Im Methanolreaktor (4) wird auf bekannte Weise Methanol erzeugt. Das Gemisch aus dem Reaktor (4) gelangt über Wärmetauscher (10) in den Dehydratisierungsreaktor (11). Zum Anfahren des Reaktors (11) kann beispielsweise ein elektrischer Vorheizer (9) eingeschaltet werden, der nach Inbetriebnahme umfahren werden kann. Das Produktgemisch gelangt nunmehr über Kühler (12) in die im allgemeinen unter Druck arbeitende Wäsche (13). Über (14) wird Waschflüssigkeit, wie beispielsweise Methanol oder Methanol/Wasser-Gemisch zugeführt. Aus dem Sumpf von (13) gelangt ein Dimethylether/Methanol/Wasser-Gemisch in Destillationskolonne (15).
Methanol kann über (16) vor den Eingang des Dehydratisierungsreaktors (11) geführt werden.

Je nach Bedarf kann das Gemisch aus (4) teilweise oder vollständig direkt nach Wärmetausch in (31) über (17) nach (13) oder nach Kühlung in (18) in den Hochdruckabscheider (19) geführt werden. (13) kann ebenfalls als Hochdruckabscheider verwendet werden. Aus letzterem kann Rohmethanol über (20) direkt zur Kolonne (21) und zur Kolonne (22) geführt werden, während das Kreislaufgas über (6) in den Methanolreaktor (4) geleitet wird.
Aus (19) kann auch Rohmethanol über (29) nach (11) gefahren werden. Schließlich kann dieses auch über (30) als Waschflüssigkeit in (13) eingesetzt werden. In (6) und (16) befinden sich Gasumlaufpumpen bzw. Kompressoren. In (15) erfolgt die Gewinnung von reinem Dimethylether über (23). Über Kopf gehendes Abgas (H$_2$, CO, N$_2$, CH$_4$ u.a.) wird in (24) mit Methanol (25) gewaschen. Der Sumpf aus (15) gelangt in Destillationskolonne (21) in der über Kopf Verunreinigungen (26) abgezogen werden. Der Sumpf aus (21) gelangt in Destillationskolonne (22), in der reines Methanol (27) gewonnen wird. Über (28) wird aus (22) Abwasser abgezogen.


## Ansprüche

1. Verfahren zur katalytischen Herstellung von Dimethylether aus Methanol über Dehydratisierungskatalysatoren bei einer Temperatur von 240 - 500 °C und einem Druck von 1 bis 350 bar und destillative Aufarbeitung des Dehydratisierungsprodukts, dadurch gekennzeichnet, daß das aus dem Methanolsynthesereaktor austretende Gemisch zumindest teilweise, ohne vorherige Abtrennung nicht umgesetzten Synthesegases und ohne Reinigung des erzeugten Methanols, in einem Dehydratisierungsreaktor zur Gewinnung von Dimethylether umgesetzt wird.

2. Verfahren nach Anspruch 1, dadurch gekennzeichnet, daß der Dehydratisierungsreaktor bei gleichem oder nur bis zu 10 bar geringerem Druck als der Methanolreaktor betrieben wird.

3. Verfahren nach den Ansprüchen 1 und 2, dadurch gekennzeichnet, daß die Wärme des aus dem Dehydratisierungsreaktor austretenden Gemischs zur Aufheizung des aus dem Methanolreaktor austretenden Gemischs durch Wärmetausch genutzt wird.

4. Verfahren nach den Ansprüchen 1 - 3, dadurch gekennzeichnet, daß der Dimethylether aus dem Gemisch, das aus dem Dehydratisierungsreaktor austritt, in einer Druckwaschapparatur ausgewaschen wird.

5. Verfahren nach den Ansprüchen 1 - 4, dadurch gekennzeichnet, daß die Druckwaschapparatur bei dem gleichen Druck bzw. nahezu dem gleichen Druck wie der Dehydratisierungsreaktor betrieben wird.

6. Verfahren nach den Ansprüchen 1 - 5, dadurch gekennzeichnet, daß als Waschflüssigkeit in der Druckwaschapparatur Methanol oder Methanol/Wasser-Gemische verwendet wird.

7. Verfahren nach den Ansprüchen 1 - 6, dadurch gekennzeichnet, daß im Gegenstrom gewaschen wird.

8. Verfahren nach den Ansprüchen 1 - 7, dadurch gekennzeichnet, daß über eine By-pass-Leitung das aus dem Methanolsynthesereaktor austretende Gemisch unter Umgehung des Dehydratisierungsreaktors über einen Wärmetauscher direkt in die Druckwaschanlage gelangt, in der die flüssige Phase vom nicht umgesetzten Synthesegas (Kreislaufgas) abgetrennt wird.

9. Verfahren nach den Ansprüchen 1 - 8, dadurch gekennzeichnet, daß Dehydratisierungskatalysatoren auf γ-Al$_2$O$_3$-Basis eingesetzt werden.

10. Verfahren nach den Ansprüchen 1 - 9, dadurch gekennzeichnet, daß der γ-Al$_2$O$_3$-Kontakt 0,0001 bis 0,5, bevorzugt 0,001 - 0,4 Gew.-% SiO$_2$ enthält.

EP 0 343 454 A2